# EUROPÄISCHE PATENTSCHRIFT

(11) **EP 2 667 906 B1**
(45) Veröffentlichungstag und Bekanntmachung des Hinweises auf die Patenterteilung: **24.05.2017**
(21) Anmeldenummer: 12702758.9
(22) Anmeldetag: 27.01.2012
(51) Int. Cl.: A61M 1/10, F04B 43/12

(54) **SCHLAUCHPUMPE UND HIERMIT AUSGESTATTETE BLUTBEHANDLUNGSVORRICHTUNG**
HOSE PUMP AND BLOOD TREATMENT DEVICE EQUIPPED THEREWITH
POMPE PÉRISTALTIQUE ET DISPOSITIF DE TRAITEMENT DU SANG ÉQUIPÉ DE LADITE POMPE

(30) Priorität: 28.01.2011 DE 102011009777; 28.01.2011 US 201161437025 P
(43) Veröffentlichungstag der Anmeldung: 04.12.2013
(73) Patentinhaber: Fresenius Medical Care Deutschland GmbH, 61352 Bad Homburg (DE)
(72) Erfinder: STEJSKAL, Martin, 65812 Bad Soden (DE); LANGKAU, Wolfram, 61449 Steinbach (DE); OESTERREICH, Stefan, 61267 Neu-Anspach (DE)
(74) Vertreter: Bobbert & Partner Patentanwälte PartmbB
(86) Internationale Anmeldenummer: PCT/EP2012/000369
(87) Internationale Veröffentlichungsnummer: WO 2012/100952

(56) Entgegenhaltungen:
- EP-A1- 1 767 232
- EP-A2- 1 469 201
- WO-A1-2009/025686
- US-A1- 2006 177 328
- US-A1- 2010 202 907

## Beschreibung

Die vorliegende Erfindung betrifft eine Schlauchpumpe gemäß Anspruch 1. Sie betrifft ferner eine Blutbehandlungsvorrichtung gemäß Anspruch 11.

Aus der medizinischen Praxis sind Schlauchpumpen bekannt. Beispiele hierzu sind in den Anmeldungen WO 2009/025686 A1, EP 1 469 201 A2, US 2010/202907 A1; US 2006/177328 A1, EP 1 767 232 A1 beschrieben. In Pumpen dieser Art wird ein Schlauch eingelegt, in welchem sich ein medizinisches flüssiges Fluid - z. B. während einer Blutbehandlung wie der Hämodialyse extrakorporal geführtes Blut - befindet. Mittels der o. g. Pumpen wird das sich im Schlauch befindende Fluid innerhalb des Schlauchs gefördert.

Eine Aufgabe der vorliegenden Erfindung ist es, eine weitere Schlauchpumpe der oben genannten Art vorzuschlagen.

Die erfindungsgemäße Aufgabe wird durch eine Schlauchpumpe mit den Merkmalen des Anspruchs 1 gelöst.

Die erfindungsgemäße Schlauchpumpe (im Folgenden auch kurz: Pumpe oder erfindungsgemäße Pumpe) dient der Aufnahme wenigstens eines Abschnitts eines Schlauchs. Die Pumpe weist wenigstens einen Stator und wenigstens einen um eine Drehachse drehbar im Stator gelagerten Rotor auf.

Der Stator umgibt oder begrenzt ganz oder teilweise den Rotor unter Aussparung zumindest von einem ersten Durchlass, vorgesehen zum Einführen des Schlauchs in die Pumpe, und einem zweiten Durchlass, vorgesehen zum Ausführen des Schlauchs aus der Pumpe heraus. Der erste und der zweite Durchlass liegen getrennt voneinander vor.

Zwischen dem ersten und dem zweiten, also den getrennt voneinander vorliegenden Durchlässen, ist ein Abschnitt angeordnet, welcher dem Stator oder einem anderen Abschnitt der Pumpe zuzurechnen ist.

Dieser Abschnitt weist in einem Bereich des zweiten Durchlasses zum Ausführen des Schlauchs einen Bereich oder einen Vorsprung auf, welcher einen geringeren Abstand zur Drehachse des Rotors aufweist als alle anderen Abschnitte des Stators.

Die erfindungsgemäße Aufgabe wird auch durch eine Blutbehandlungsvorrichtung mit den Merkmalen des Anspruchs 11 gelöst. Diese weist wenigstens eine erfindungsgemäße Schlauchpumpe auf.

Vorteilhafte Weiterentwicklungen und spezielle Ausgestaltungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll bestimmte erfindungsgemäße Ausführungsformen erläutern.

Der Abstand des wenigstens einen Abschnitts zur Drehachse des Rotors wird - wie auch die Abstände der übrigen Abschnitte - in bestimmten erfindungsgemäßen Ausführungsformen jeweils senkrecht zur Drehachse bestimmt. Unter "Drehachse" ist der geometrische Ort einer in ihrer Ausdehnung nicht beschränkten Geraden zu verstehen.

Der Vorsprung erstreckt sich in manchen erfindungsgemäßen Ausführungsformen in Richtung zum Rotor, und/oder zur Drehachse, und/oder radial nach innen, und/oder zu einem Inneren der Blutpumpe.

In bestimmten erfindungsgemäßen Ausführungsformen umgibt oder begrenzt der Stator und/oder der zwischen dem ersten und dem zweiten Durchlass angeordnete Abschnitt den Rotor in einer Ebene senkrecht zur Drehachse oder in einer Drehebene des Rotors und/oder in einer zur Drehebene parallelen Ebene.

Die Anzahl der Aussparungen beträgt wenigstens zwei. Sie kann höher liegen; z. B. können drei oder vier oder mehr Aussparungen vorgesehen sein.

Die Aussparungen können ergänzend solche umfassen, durch welche kein Schlauch in den Raum zwischen Stator und Rotor ein- oder aus diesem austritt.

Alle Aussparungen sind in bestimmten erfindungsgemäßen Ausführungsformen getrennt durch Statormaterial oder Material, welches einstückig mit dem Stator verbunden ist. In anderen erfindungsgemäßen Ausführungsformen sind alle Aussparungen mittels des Materials der erfindungsgemäßen Pumpe voneinander getrennt, welches nicht Teil des Stators ist. In gewissen erfindungsgemäßen Ausführungsformen sind die Aussparungen getrennt sowohl durch Material des Stators als auch durch Material, welches nicht Teil des Stators ist.

Der erste Durchlass liegt in bestimmten erfindungsgemäßen Ausführungsformen stromaufwärts zum zweiten Durchlass, bezogen auf die Strömungsrichtung des im Schlauch strömenden Fluids in einer Normalanwendung der Pumpe. Anders ausgedrückt erreicht das im Schlauch geförderte Fluid zunächst den ersten Durchlass und später erst den zweiten Durchlass.

In bestimmten erfindungsgemäßen Ausführungsformen ist die Pumpe als peristaltische Pumpe und/oder als Rollenpumpe ausgestaltet.

In manchen erfindungsgemäßen Ausführungsformen ist die Pumpe zum Fördern einer Lösung zur Antikoagulation vorgesehen, insbesondere zum Fördern von Citrat- oder Calciumlösungen, ferner Heparinlösungen. In einigen erfindungsgemäßen Ausführungsformen ist die Pumpe als Blutschlauchpumpe zum Fördern von Blut vorgesehen. In bestimmten erfindungsgemäßen Ausführungsformen ist die Pumpe zum Fördern von Dialysat, Filtrat, Substituat oder dergleichen vorgesehen. Die erfindungsgemäße Pumpe und/oder die erfindungsgemäße Blutbehandlungsvorrichtung kann jeweils die der Verwendung entsprechenden Schaltungen zur Steuerung oder Regelung aufweisen.

In einigen erfindungsgemäßen Ausführungsformen weist die Pumpe kein von der Pumpe lösbares Abdeckelement zum Verhindern eines Verfangens oder Verletzens an den Eintritts- oder Austrittsstellen auf, an denen der Schlauch in die Pumpe ein- oder aus dieser austritt.

In manchen erfindungsgemäßen Ausführungsformen weist die Pumpe kein Abdeckelement zum Verhindern eines Verfangens oder Verletzens an den Eintritts- oder Austrittsstellen auf, welches nicht einstückig mit dem Stator und/oder dem Gehäuse der Pumpe ist, oder einstückig mit dem Stator und/oder dem Gehäuse der Pumpe vorliegt.

In bestimmten erfindungsgemäßen Ausführungsformen ist ein Schutz vor Verfangen oder Verletzen allein durch die Ausgestaltung und/oder das Zusammenwirken von Rotor und/oder Stator und ihrer Komponenten bewirkt.

In manchen erfindungsgemäßen Ausführungsformen überragen die den Rotor umgebenden Abschnitte des Stators den Rotor in einer Richtung entlang der Drehachse aus der Pumpe heraus oder decken diese ab, oder sind mit diesen bündig (insbesondere bündig in einer Richtung aus der Pumpe heraus). Nicht überragt oder abgedeckt ist in einigen erfindungsgemäßen Ausführungsformen allein eine Rotorabdeckung, welche optional zusätzlich Rotorgriffe aufweist. In diesen einigen erfindungsgemäßen Ausführungsformen sind alle übrigen Komponenten des Rotors vom Stator überdeckt.

Bei den Abschnitten des Stators, welche den Rotor umgeben, handelt es sich in bestimmten erfindungsgemäßen Ausführungsformen wenigstens um die Abschnitte des Stators, welche im Gebrauch der Schlauchpumpe mit dem Schlauch in Kontakt kommen oder Gegenlager hierfür sind. In einigen erfindungsgemäßen Ausführungsformen zählt hierzu der Abschnitt, welcher einen geringeren Abstand zur Drehachse des Rotors aufweist und/oder jener Abschnitt, welcher zwischen den beiden Durchlässen für den Schlauch liegt.

In bestimmten erfindungsgemäßen Ausführungsformen überdecken die Abschnitte des Stators, welche den Rotor umgeben, zumindest die im Gebrauch der Pumpe rotierenden Abschnitte des Rotors oder schirmen diese, z. B. gegen Berührung, vorteilhaft ab.

In einigen erfindungsgemäßen Ausführungsformen ist der Bereich oder Vorsprung mit dem geringeren Abstand zur Drehachse derart vorgesehen, dass er weder Abschnitte des Rotors berührt noch in Kontakt steht mit anderen Abschnitten des Stators als jenem, welcher zwischen dem ersten und dem zweiten Durchlass vorliegt.

In gewissen erfindungsgemäßen Ausführungsformen steht der Bereich oder Vorsprung nasenartig oder nasenförmig vom Abschnitt vor, welcher zwischen dem ersten und dem zweiten Durchlass vorliegt.

In bestimmten erfindungsgemäßen Ausführungsformen der Pumpe sind der erste und/oder der zweite Durchlass bei Blick von vorne auf die Pumpe nach vorne offen. Eine Abdeckung, die den Blick auf den durch den ersten und/oder durch den zweiten Durchlass geführten Schlauch während des Gebrauchs der Pumpe verhindern würde, ist in diesen Ausführungsformen nicht vorgesehen. Dies erlaubt ein vorteilhaft einfaches Einlegen und Entnehmen des Schlauchs in die bzw. aus der Pumpe. Es kann ferner vorteilhaft dazu beitragen, die Kosten und den Aufwand für Herstellung und Montage der Pumpe niedrig zu halten.

In manchen erfindungsgemäßen Ausführungsformen ist der Bereich oder Vorsprung mit dem geringeren Abstand zur Drehachse in einem Bereich des zwischen den getrennt voneinander vorliegenden Durchlässen ausgestalteten Abschnitts ausgestaltet, welcher im Gebrauch der Pumpe nicht im Laufweg von Fluidfortbewegungseinrichtungen (wie z. B. Rollen) des Rotors liegt.

In einigen erfindungsgemäßen Ausführungen der Schlauchpumpe dient der Bereich oder Vorsprung mit dem geringeren Abstand zur Drehachse nicht dazu, eine Umlenkung des Schlauchs in eine gewünschte Richtung zu ermöglichen.

In manchen erfindungsgemäßen Ausführungsformen der Schlauchpumpe berührt der in die Schlauchpumpe eingelegte Schlauch den Bereich oder Vorsprung nicht.

Erfindungsgemäß befindet sich der Bereich oder Vorsprung nicht in einer Laufbahn von Verdrängungskörpern oder Rollen der Schlauchpumpe oder ihres Rotors.

In bestimmten erfindungsgemäßen Ausführungsformen bedeutet "nicht im Laufweg von Fluidfortbewegungseinrichtungen", dass der Bereich oder Vorsprung mit dem geringeren Abstand zur Drehachse im Normalbetrieb keinen Kontakt zu einer Lauffläche am Stator und/oder keinen Kontakt mit dem Schlauch hat. Dies erlaubt es vorteilhaft, die Abmessungen der Pumpe - zumindest in einer Einbautiefe oder einer Erstreckung in Richtung der Drehachse - gering zu halten. Die Pumpe steht in dieser Ausgestaltung vorteilhafterweise nur wenig über z. B. eine Vorderfront der erfindungsgemäßen Blutbehandlungsvorrichtung hinaus.

In manchen erfindungsgemäßen Ausführungsformen sind die Fluidfortbewegungseinrichtungen als Rollen ausgestaltet.

In bestimmten erfindungsgemäßen Ausführungsformen verjüngt sich der Bereich oder Vorsprung mit dem geringeren Abstand zur Drehachse in einer Ebene senkrecht zur Drehachse in einer Richtung entgegen der vorgesehenen Drehrichtung des Rotors zur Spitze des Abschnitts hin oder zu seinem Ende hin.

Das Ende des Bereichs oder Vorsprungs kann das in Drehrichtung des Rotors im Normalbetrieb nähere Ende sein.

In manchen erfindungsgemäßen Ausführungsformen ist der Abschnitt mit dem geringeren Abstand zur Drehachse zu seinem Ende hin spitzer oder zugespitzt (beispielsweise nimmt ein Winkel, mit dem Außenkonturen aufeinander zulaufen, ab). In einigen erfindungsgemäßen Ausführungsformen hat dieser Abschnitt eine Keilform, wobei die Spitze des Keils entgegen der Drehrichtung des Rotors zeigt.

In einigen erfindungsgemäßen Ausführungsformen findet man die Spitze, das Zulaufen oder die Keilform in einer Schnittebene durch den Abschnitt mit dem geringen Abstand zur Drehachse in einer Ebene senkrecht zur Drehachse.

Erfindungsgemäß weist der Rotor eine Rotorabdeckung auf, die im Gebrauch der Pumpe gemeinsam mit dem Rotor rotiert. Die Rotorabdeckung kann ein Griffstück mit oder ohne Griffflügeln aufweisen, muss ein solches jedoch nicht aufweisen. Der Bereich oder der
Vorsprung, welcher einen geringeren Abstand zur Drehachse des Rotors aufweist, ist in diesen Ausführungsformen derart an der Pumpe angeordnet, dass ein Spalt, welcher in einer radialen Richtung zwischen der rotierenden Rotorabdeckung und dem Bereich oder Vorsprung mit dem geringeren Abstand liegt, eine vorbestimmte Breite nicht übersteigt.

In einigen erfindungsgemäßen Ausführungsformen entspricht die vorbestimmte Breite einer beliebigen Breite, welche geringer ist als jede Breite von Spalten zwischen der Rotorabdeckung (oder einem Rand hiervon) und anderen Abschnitten, welche den Rotor umgeben. Ob dieses Kriterium erfüllt ist, kann beispielsweise in einer Ebene senkrecht zur Drehachse ermittelt werden, vorzugsweise in einer Ebene, in welcher auch die Rotorabdeckung liegt.

In manchen erfindungsgemäßen Ausführungsformen ist die vorbestimmte Breite des Spalts jede Breite, die geringer ist als ein Durchmesser eines mit der Pumpe verwendeten oder zur Verwendung vorgesehenen Schlauchs. Der Durchmesser kann dabei jener sein, den der Schlauch vor dessen Gebrauch einnimmt. Es kann jener Durchmesser sein, den der Schlauch während seines Gebrauchs einnimmt, wahlweise während Fluidfortbewegungseinrichtungen auf ihn wirken, oder während diese nicht auf ihn wirken.

In einigen erfindungsgemäßen Ausführungsformen ist die Breite des Spalts geringer als ein mittlerer oder gemittelter Abstand zwischen einer von Fluidfortbewegungseinrichtungen (z. B. Rollen) des Rotors bei rotierendem Rotor umschriebener Kreisbahn und einem mittleren oder gemittelten Innendurchmesser des Stators.

In manchen erfindungsgemäßen Ausführungsformen ist die Breite des Spalts geringer als ein kleinster Abstand zwischen einer von Fluidfortbewegungseinrichtungen (z. B. Rollen) des Rotors bei rotierendem Rotor umschriebener Kreisbahn und einem größten Innendurchmesser des Stators.

In manchen erfindungsgemäßen Ausführungsformen weist der Rotor eine Grundplatte auf, welche bei Blick von vorne auf die Pumpe hinter dem Schlauch liegt. In einigen erfindungsgemäßen Ausführungsformen ist die Grundplatte an wenigstens einem Abschnitt hiervon abgeflacht, ausgeschnitten oder abgeschnitten derart, dass - oder um - ein Herausnehmen des Rotors aus dem Stator in Richtung der Drehachse des Rotors nach vorne aus der Pumpe heraus erlaubt wird bzw. zu erlauben oder vereinfacht wird bzw. zu vereinfachen.

In bestimmten erfindungsgemäßen Ausführungsformen ist der abgeflachte, ausgeschnittene oder abgeschnittene Abschnitt dabei derart ausgestaltet, dass ein Herausnehmen des Rotors aus der Pumpe mittels Bewegens des Rotors allein in einer Richtung entlang der Drehachse möglich ist. Dies erlaubt vorteilhaft ein Herausnehmen ohne Verkanten oder Verdrehen des Rotors. Letzteres erleichtert das Herausnehmen vorteilhaft und beugt Beschädigungen vor, die aufgrund des Herausnehmens entstehen könnten.

In manchen erfindungsgemäßen Ausführungsformen ist der zwischen den getrennt voneinander vorliegenden Durchlässen ausgestaltete Abschnitt des Stators in einem Bereich des ersten Durchlasses in einer Ebene senkrecht zur Drehachse des Rotors in der vorgesehenen Drehrichtung des Rotors zu seiner Spitze oder zu seinem (in Durchströmungsrichtung des Schlauchs hinterem) Ende hin verjüngt, spitz oder zugespitzt verlaufend oder keilförmig ausgestaltet. Diese Ausgestaltung kann zusätzlich ein Verfangen oder Verletzen am ersten Durchlass zum Einführen des Schlauchs verhindern oder unwahrscheinlicher machen.

In bestimmten erfindungsgemäßen Ausführungsformen ist die Blutbehandlungsvorrichtung eine
Hämodialysevorrichtung, eine Hämofiltrationsvorrichtung, eine Hämodiafiltrationsvorrichtung, Apheresevorrichtung, Transfusionsvorrichtung oder Oxygenierungsvorrichtung.

In manchen erfindungsgemäßen Ausführungsformen ist die Wandung des Stators und/oder die Wandung des zwischen den Durchlässen vorgesehenen Abschnitts mit einer Höhe oder Erstreckung (in einer Richtung parallel zur Drehachse) derart ausgestaltet, dass die Wandung den Rotor (abgesehen allenfalls vom Rotorgriff) radial vollständig umgibt oder abschirmt. Auf diese Weise verbleibt vorteilhaft kaum eine Stelle, an welcher ein Verfangen oder gar ein Verletzen erfolgen kann. Dies kann allenfalls an den Durchlässen für Schlaucheintritt oder Schlauchaustritt passieren, falls überhaupt.

Eine eigenständige Abdeckung des Spalts zwischen Rotor und Stator bzw. der bekannten Fangstellen durch Anbringen einer isoliert hergestellten und in einem eigenen Arbeitsschritt angebrachten Abdeckung kann aufgrund der besonderen Ausgestaltung der Pumpe in einigen Ausführungsformen erfindungsgemäß entfallen. Dies erlaubt vorteilhaft, Kosten und Aufwand bei Herstellung und Montage zu verringern.

In bestimmten erfindungsgemäßen Ausführungsformen kann somit auf zusätzliche Bauteile wie eine Abdeckung zum Schutz vor Verfangen oder Verletzen vorteilhaft verzichtet werden. Dies gilt insbesondere dann, wenn der Stator und/oder das Gehäuse der Pumpe, jeweils einschließlich des oben diskutierten Vorsprungs, welcher/welches einen geringen Abstand zur Drehachse hat, in einem Gießverfahren, z. B. einem Spritzgussverfahren, hergestellt wurde.

Ein wie hierin beschriebenes Abdecken oder Verengen durch den Vorsprung oder Bereich, der einen geringeren Abstand zur
Drehachse des Rotors aufweist als andere Abschnitte des Rotors, stellt sicher oder trägt dazu bei, dass beispielsweise Verletzungen, die durch das Drehen des Rotors von Hand beim Einlegen des Schlauchs in die Schlauchpumpe in diesem Bereich entstehen könnten, vorteilhaft vermieden werden können.

Die vorliegende Erfindung wird im Folgenden anhand der beigefügten Zeichnungen, in welchen identische Bezugszeichen gleiche oder ähnliche Bauteile bezeichnen, exemplarisch erläutert. In den Figuren gilt:
- **Fig. 1**: zeigt die erfindungsgemäße Schlauchpumpe einer exemplarischen Ausführungsform perspektivisch von vorne rechts;
- **Fig. 2**: zeigt die Schlauchpumpe der Fig. 1 von vorne;
- **Fig. 3**: zeigt die Schlauchpumpe der Fig. 1 und 2 perspektivisch von unten rechts;
- **Fig. 4**: zeigt die Schlauchpumpe der vorangegangenen Figuren von unten;
- **Fig. 5**: zeigt die Schlauchpumpe der vorangegangenen Figuren von rechts; und
- **Fig. 6**: zeigt die Schlauchpumpe der vorangegangenen Figuren von hinten.

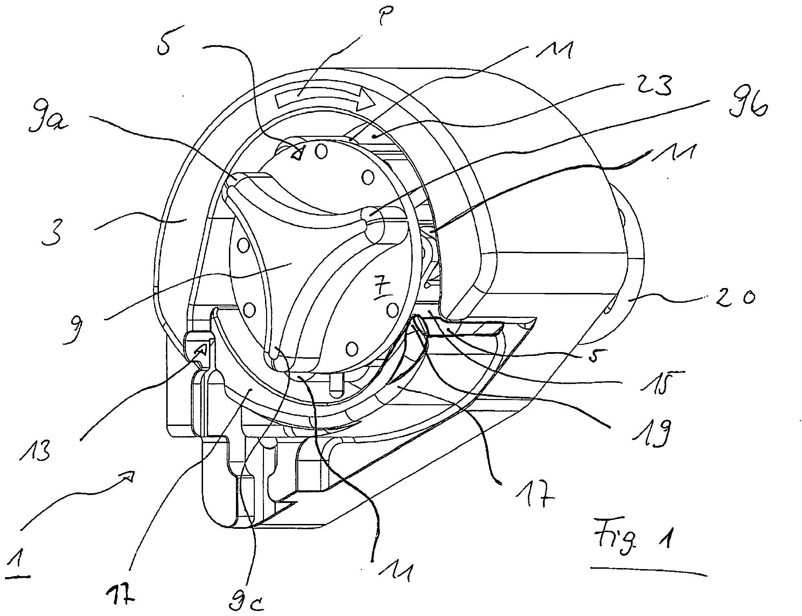

**Fig. 1** zeigt eine erfindungsgemäße Schlauchpumpe oder Pumpe 1 in einer exemplarischen Ausführungsform perspektivisch von vorne rechts.

Die Pumpe 1 weist einen in ihr Gehäuse integrierten bzw. mit diesem einstückig hergestellten Stator 3 auf. In einem Inneren des Stators 3 kann ein Rotor 5 um eine in Fig. 1 nicht markierte Drehachse drehen. Der Rotor 5 weist eine als Rotorabdeckung 7 ausgestaltete vordere, rotierende Abdeckplatte oder -scheibe auf. Die Rotorabdeckung 7 weist einen - exemplarisch dreiflügelig ausgestalteten - Rotorgriff 9 mit drei Griffflügeln 9a, 9b, 9c zum manuellen Drehen des Rotors 5 auf. Der Rotor 5 weist Rollen 11 als Fluidfortbewegungseinrichtungen auf. Im hier betrachteten Betriebszustand (entspricht dem Normalbetrieb) dreht der Rotor 5 in Richtung des Pfeils P.

Zwischen Stator 3 und Rotor 5 verbleibt erkennbar ein Spalt, in welchen bei Gebrauch der Pumpe 1 ein Abschnitt eines in den Figuren nicht gezeigten Schlauchs eingelegt wird.

Der Schlauch kann zu seinem Einlegen in den Spalt, welcher sich zwischen Stator 3 und Rotor 5 befindet, durch einen ersten Durchlass 13 in das Innere der Pumpe 1 hineingeführt werden. Der Schlauch kann nach Vollziehen eines Dreiviertelkreises (geschätzt) schließlich durch einen zweiten Durchlass 15 wieder aus dem Inneren der Pumpe 1 herausgeführt werden.

Zwischen dem ersten Durchlass 13 und dem zweiten Durchlass 15 liegt ein Abschnitt 17, welcher beide Durchlässe 13 und 15 räumlich voneinander trennt.

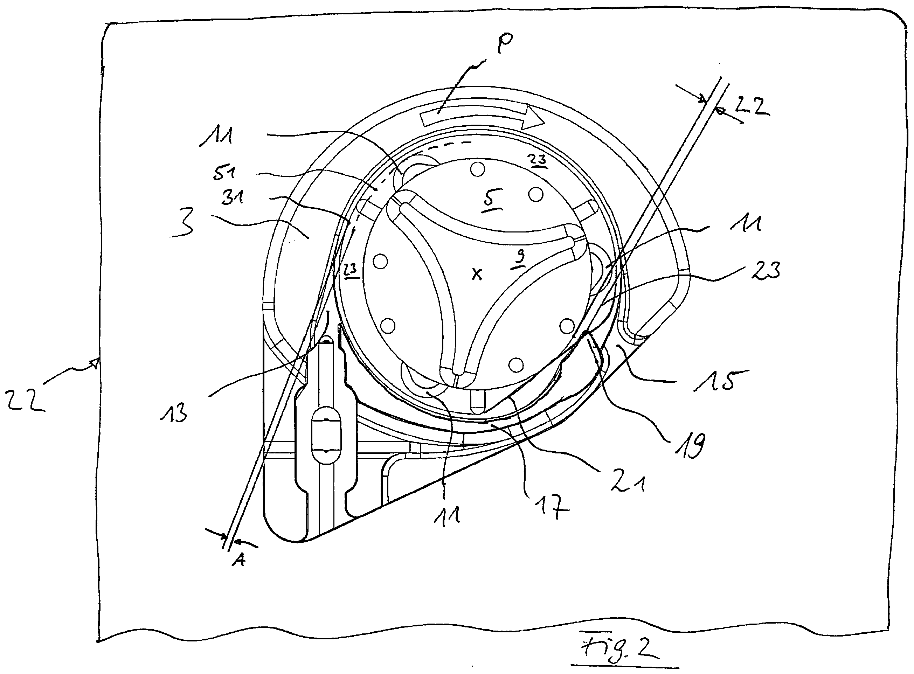

Der Abschnitt 17 kann Teil des Stators 3 und/oder Teil eines anderen Abschnitts der Schlauchpumpe 1 sein. Der Abschnitt 17 weist einen - in einer Richtung parallel zur nicht markierten Drehachse des Rotors 5 dünn ausgestalteten - Vorsprung 19 auf, welcher einen geringeren Abstand zur nicht dargestellten Drehachse des Rotors 5 aufweist als andere Abschnitte des Stators 3. Sowohl diese Beziehung als auch der Vorsprung 19 sind in Fig. 2 noch besser zu erkennen.

Eine Antriebseinheit, beispielsweise ausgestaltet als Gleichstrom- oder Wechselstrommotor, zum Antreiben des Rotors 5 ist mit dem Bezugszeichen 20 bezeichnet.

**Fig. 2** zeigt die Schlauchpumpe 1 der Fig. 1 von vorne. Der Vorsprung 19 verläuft abweichend vom übrigen Verlauf des Abschnitts 17. Er ragt näher an die nicht dargestellte Drehachse, welche sich durch die mit "x" bezeichnete Stelle in die Tiefe der Zeichenebene hinein und aus dieser heraus erstreckt, als alle anderen Abschnitte des Stators 3.

In Fig. 2 ist ein Spalt 22 markiert, welcher in einer radialen Richtung zwischen der rotierenden Rotorabdeckung 7 und dem Vorsprung 19 mit dem geringeren Abstand liegt. Seine Breite ist erkennbar geringer als die Breite eines beliebigen anderen Abstands zwischen Rotor 5 einerseits und Stator 3 bzw. Abschnitt 17 anderseits innerhalb der Zeichenebene (also in einer Ebene senkrecht zur Drehachse).

In Fig. 2 ist eine Abflachung 21 einer Grundplatte 23 des Rotors 5 zu erkennen. Die Abflachung 21 erlaubt vorteilhaft ein Einsetzen oder Herausnehmen des Rotors 5 in das oder aus dem Stator 3 bzw. in das oder aus dem Gehäuse 25 der Pumpe 1, beispielsweise zu Wartungszwecken.

Fig. 2 zeigt einen mittlerer Abstand A zwischen einer von den Rollen 11 des Rotors 5 bei rotierendem Rotor 5 umschriebenen Kreisbahn 51 (in Fig. 2 als Strichlinie angedeutet) und einem mittleren Innendurchmesser 31 - welcher im Normalgebrauch Kontakt mit dem Schlauch hat - des Stators 3.

In Fig. 2 ist ferner eine nur teilweise dargestellte, erfindungsgemäße Behandlungsvorrichtung 24 höchst schematisch angedeutet.

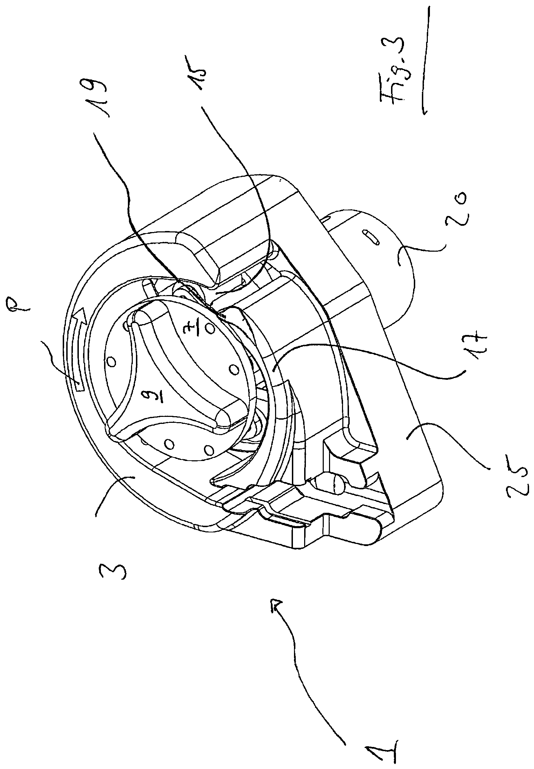

**Fig. 3** zeigt die Schlauchpumpe 1 der vorangegangenen Figuren perspektivisch von unten rechts.

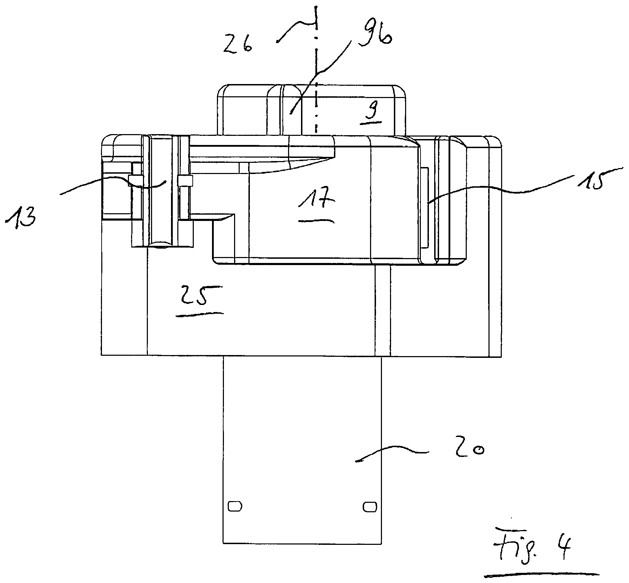

**Fig. 4** zeigt die Schlauchpumpe 1 der vorangegangenen Figuren von unten.

Fig. 4 zeigt deutlich, dass die Wandung sowohl des Stators 3 als auch des zwischen den Durchlässen 13 und 15 gelegenen Abschnitts 17 in Richtung zum Rotorgriff 9 derart hochgezogen ausgestaltet ist, dass die Wandung den Rotor 5 - abgesehen nur vom Rotorgriff 9 - vollständig umgibt oder abschirmt.

Die Drehachse des Rotors 5 ist mit dem Bezugszeichen 26 bezeichnet.

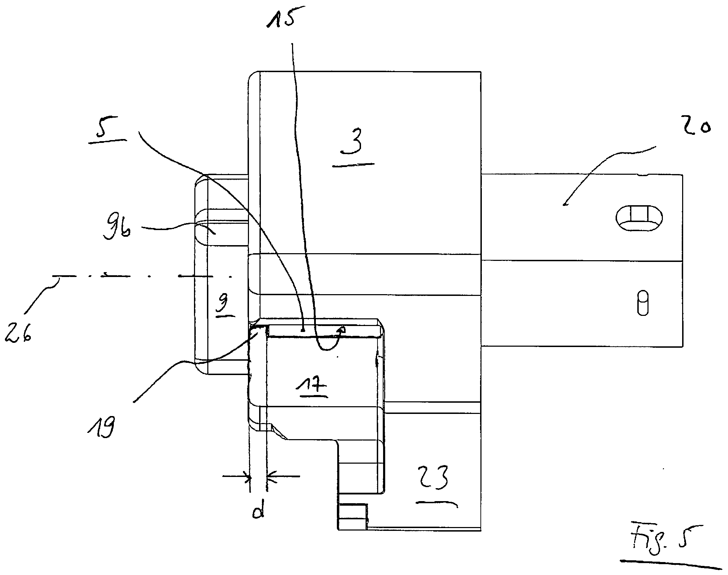

**Fig. 5** zeigt die Schlauchpumpe 1 der vorangegangenen Figuren von rechts.

Fig. 5 zeigt deutlich, dass der Vorsprung 19 in einer Richtung parallel zur Drehachse eine nur geringe Erstreckung "d" hat. Der Vorsprung 19 ist auf diese Weise derart dünn ausgestaltet, dass in Fig. 5 durch den zweiten Durchlass 15 hindurch der Blick freigegeben ist auf Teile des Rotors 5. Eine Laufbahn für die in Fig. 5 nicht gezeigten Rollen des Rotors 5 ist daher durch den Vorsprung 19 vorteilhaft nicht beeinträchtigt.

In bestimmten erfindungsgemäßen Ausführungsformen entspricht die Erstreckung "d" der Dicke der Rotorabdeckung 7.

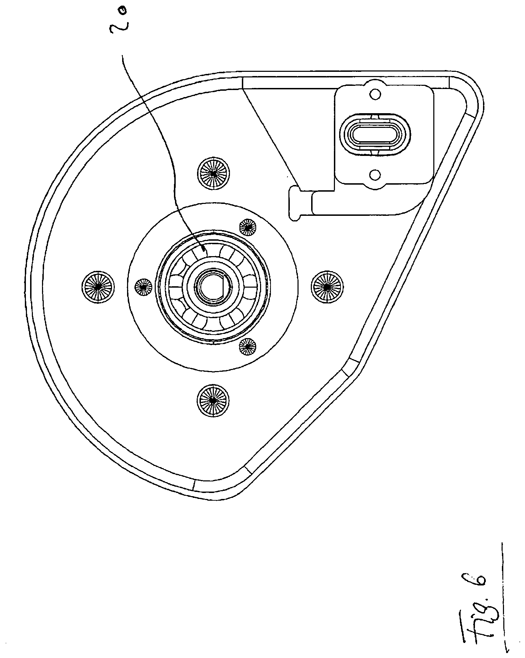

**Fig. 6** zeigt die Schlauchpumpe 1 der vorangegangenen Figuren von hinten.

### Bezugszeichenliste

- 1: Pumpe
- 3: Stator
- 31: mittlerer Innendurchmesser des Rotors
- 5: Rotor
- 51: von Fluidfortbewegungseinrichtungen des Rotors bei rotierendem Rotor umschriebene Kreisbahn
- 7: Rotorabdeckung
- 9: Rotorgriff
- 9a-c: Griffflügel
- 11: Rollen
- 13: erster Durchlass
- 15: zweiter Durchlass
- 17: Abschnitt
- 19: Vorsprung
- 20: Antriebseinheit
- 21: Abflachung
- 22: Spalt
- 23: Grundplatte
- 24: Behandlungsvorrichtung
- 25: Gehäuse
- 26: Drehachse
- A: Abstand
- P: Pfeil
- d: Erstreckung

## Patentansprüche

1. Schlauchpumpe (1) zur Aufnahme wenigstens eines Abschnitts eines Schlauchs mit wenigstens einem Stator (3) und wenigstens einem um eine Drehachse (26) drehbar im Stator (3) gelagerten Rotor (5), wobei der Rotor (5) eine als Rotorabdeckung (7) ausgestaltete vordere, rotierende Abdeckplatte oder -scheibe mit einem Rotorgriff (9) zum manuellen Drehen des Rotors aufweist, wobei der Stator (3) den Rotor (5) unter Aussparung eines ersten Durchlasses (13) zum Einführen des Schlauchs in die Schlauchpumpe (1) hinein und eines hiervon getrennt vorliegenden zweiten Durchlasses (15) zum Ausführen des Schlauchs aus der Schlauchpumpe (1) heraus zumindest in Abschnitten des Umfangs des Rotors (5) umgibt oder diesen begrenzt, **dadurch gekennzeichnet,**
- **dass** ein zwischen dem ersten Durchlass (13) und dem zweiten Durchlass (15) ausgestalteter Abschnitt (17) der Schlauchpumpe (1) oder des Stators (3) in einem Bereich des zweiten Durchlasses (15) einen Bereich oder Vorsprung (19) aufweist, welcher einen geringeren Abstand zur Drehachse (26) des Rotors (5) aufweist als alle anderen Abschnitte des Stators (3),
- wobei der Bereich oder Vorsprung (19) sich nicht in einer Laufbahn von Verdrängungskörper oder Rollen (11) der Schlauchpumpe (1) oder ihres Rotors (5) befindet.

2. Schlauchpumpe (1) nach Anspruch 1, wobei die den Rotor (5) umgebenden oder begrenzenden Abschnitte des Stators (3) oder der zwischen dem ersten Durchlass (13) und dem zweiten Durchlass (15) ausgestaltete Abschnitt (17) den Rotor (5) in einer Richtung parallel zur Drehachse (26) des Rotors (5) überragen oder wenigstens abdecken.

3. Schlauchpumpe (1) nach Anspruch 1 oder 2, wobei der Bereich oder Vorsprung (19), welcher einen geringeren Abstand zur Drehachse (26) des Rotors (5) aufweist, in einem Bereich des zwischen den getrennt voneinander vorliegenden Durchlässen (13, 15) ausgestalteten Abschnitts (17) ausgestaltet ist, welcher im Gebrauch der Schlauchpumpe (1) nicht im Laufweg von Fluidfortbewegungseinrichtungen des Rotors (5) liegt.

4. Schlauchpumpe (1) nach wenigstens einem der Ansprüche 1 bis 3, wobei sich der Bereich oder Vorsprung (19), welcher einen geringeren Abstand zur Drehachse (26) des Rotors (5) aufweist, in einer Ebene senkrecht zur Drehachse (26) und in einer Richtung entgegen der vorgesehenen Drehrichtung des Rotors (5) zu seiner Spitze oder zu seinem Ende hin verjüngt.

5. Schlauchpumpe (1) nach wenigstens einem der vorangegangenen Ansprüche, wobei der Rotor (5) eine im Gebrauch mit diesem rotierende Rotorabdeckung (7) aufweist, und wobei der Bereich oder Vorsprung (19), welcher einen geringeren Abstand zur Drehachse (26) des Rotors (5) aufweist, derart an der Schlauchpumpe (1) angeordnet ist, dass die Breite eines Spalts (22), welcher zwischen der rotierenden Rotorabdeckung (7) und dem Bereich oder Vorsprung (19) mit dem geringeren Abstand liegt, eine vorbestimmte Breite nicht übersteigt.

6. Schlauchpumpe (1) nach Anspruch 5, wobei die vorbestimmte Breite des Spalts (22) jede Breite ist, welche geringer ist als jeder Abstand, welcher zwischen anderen Abschnitten des Stators (3) und dem Rotor (5) in einer Ebene senkrecht zur Drehachse (26) besteht.

7. Schlauchpumpe (1) nach Anspruch 5 oder 6, wobei die vorbestimmte Breite des Spalts (22) jede Breite ist, welche geringer ist als ein Durchmesser eines mit der Schlauchpumpe (1) verwendeten oder zur Verwendung vorgesehenen Schlauchs vor oder während dessen Gebrauch (s).

8. Schlauchpumpe (1) nach einem der Ansprüche 5, 6 oder 7, wobei die vorbestimmte Breite des Spalts (22) jede Breite ist, welche geringer ist als ein mittlerer Abstand (A) zwischen einer von Fluidfortbewegungseinrichtungen des Rotors (5) bei rotierendem Rotor (5) umschriebenen Kreisbahn (51) und einem mittleren Innendurchmesser (31) des Stators (3).

9. Schlauchpumpe (1) nach wenigstens einem der vorangegangenen Ansprüche, wobei der Rotor (5) eine Grundplatte (23) aufweist, welche in wenigsten einem Abschnitt hiervon abgeflacht, ausgeschnitten oder abgeschnitten ist, um ein Herausnehmen des Rotors (5) aus dem Stator (3) in Richtung der Drehachse (26) des Rotors (5) zu erlauben.

10. Schlauchpumpe (1) nach wenigstens einem der vorangegangenen Ansprüche, wobei der zwischen den getrennt voneinander vorliegenden Durchlässen (13, 15) ausgestaltete Abschnitt (17), oder ein Teilabschnitt hiervon, in einem Bereich des ersten Durchlasses (13) zum Einführen in einer Ebene senkrecht zur Drehachse (26) des Rotors (5) in der vorgesehenen Drehrichtung des Rotors (5) zu seiner Spitze oder zu seinem Ende hin verjüngt oder zugespitzt ausgestaltet ist.

11. Blutbehandlungsvorrichtung (24), welche wenigstens eine Schlauchpumpe (1) gemäß wenigstens einem der Ansprüche 1 bis 10 aufweist.

12. Blutbehandlungsvorrichtung (24), nach Anspruch 11, ausgestaltet als Hämodialysevorrichtung, Hämofiltrationsvorrichtung, Hämodiafiltrationsvorrichtung, Apheresevorrichtung, Transfusionsvorrichtung oder Oxygenierungsvorrichtung.

## Claims

1. Flexible tubing pump (1) for accommodation of at least one section of a flexible tubing with at least one stator (3) and at least one rotor (5) which pivots around an axis of rotation (26) in the stator (3), wherein the rotor (5) comprises a rotating front cover or disk configured as rotor cover (7) with a rotor handle (9) to manually turn the rotor, wherein the stator (3) surrounds or confines the rotor (5) in at least some sections of the circumference of the rotor (5), by means of a recessed first opening (13) intended for inserting the flexible tubing into the pump (1), and a therefrom separately located recessed second opening (15) intended for leading the flexible tubing out of the tubing pump (1), **characterized in that**
- a section (17) of the tubing pump (1) or of the stator (3) formed between the first opening (13) and the second opening (15) comprises an edge or an area (19) in proximity of the second opening (15) which is nearer to the axis of rotation (26) of the rotor (5) than all other sections of the stator (3),
- wherein the edge or area (19) is not in the raceway of fluid movement devices or rollers (11) of the tubing pump (1) or of its rotor (5).

2. Flexible tubing pump (1) according to claim 1, wherein the sections of the stator (3) that surround or confine the rotor (5) or the section (17) formed between the first opening (13) and the second opening (15) stick(s) out or at least cover(s) the rotor (5) in a direction parallel to the axis of rotation (26) of the rotor (5).

3. Flexible tubing pump (1) according to claim 1 or claim 2, wherein the area or edge (19) having a shorter distance to the axis of rotation (26) of the rotor (5) is formed in a region of the section (17) which is formed between the spaced apart openings (13, 15) that is not in the raceway of fluid movement devices of the rotor (5) during use of the pump (1).

4. Flexible tubing pump (1) according to at least one of claims 1 to 3, wherein the area or edge (19) having a shorter distance to the axis of rotation (26) of the rotor (5) gets narrower towards its end or its tip in a plane perpendicular to the axis of rotation (26) and in a direction opposite to the rotating direction of the axis of rotation (26).

5. Flexible tubing pump (1) according to at least one of the preceding claims, wherein the rotor (5) has a rotor cover (7) which rotates simultaneously with the rotor during use and wherein the area or edge (19) having a shorter distance to the axis of rotation (26) of the rotor (5) is arranged at the flexible tubing pump (1) such that the width of a gap (22) lying between the rotating rotor cover (7) and the area or edge (19) with the shorter distance does not exceed a pre-determined width.

6. Flexible tubing pump (1) according to claim 5, wherein the pre-determined width of the gap (22) is any width smaller than any distance between other sections of the stator (3) and the rotor(5) in a plane perpendicular to the axis of rotation (26).

7. Flexible tubing pump (1) according to claim 5 or 6, wherein the pre-determined width of the gap (22) is any width smaller than a diameter of a flexible tube used with or intended to be used with the flexible tubing pump (1) before or during its use.

8. Flexible tubing pump (1) according to claim 5, 6 or 7, wherein the pre-determined width of the gap (22) is any width smaller than an average distance (A) between a circular raceway (51) described by fluid movement devices of the rotor (5) during rotation of the rotor (5) and an average inner diameter (31) of the stator (3).

9. Flexible tubing pump (1) according to at least one of the preceding claims, wherein the rotor (5) has a baseplate (23) which is flattened, cut or cut off in at least one section thereof to allow a removal of the rotor (5) from the stator (3) in the direction of the axis of rotation (26) of the rotor (5).

10. Flexible tubing pump (1) according to at least one of the preceding claims, wherein the area (17) formed between the spaced apart openings (13, 15), or segment thereof, is formed so that it gets narrower towards its end or its tip in a region of the first opening (13) for insertion in a plane perpendicular to the axis of rotation (26) of the rotor (5) in the intended direction of rotation of the rotor (5).

11. Blood treatment device (24) comprising at least one flexible tubing pump (1) according to at least one of the claims 1 to 10.

12. Blood treatment device (24) according to claim 11, configured as device for haemodialysis, hemofiltration, hemodiafiltration, apheresis, transfusion or oxygenation.

## Revendications

1. Une pompe à tuyau souple (1) pour la réception d'au moins une section d'un tuyau souple, comprenant au moins un stator (3) et au moins un rotor (5) pivotant autour d'un axe de rotation (26) dans le stator (3), dans laquelle le rotor (5) comprend comme protection de rotor (7) une plaque avant couvrante rotative, ou un disque, avec une poignée de rotor (9) pour faire tourner manuellement le rotor (5), dans laquelle le stator (3) entoure ou confine le rotor (5), ou tout au moins certaines sections de la circonférence du rotor (5), au moyen de l'évidement d'une première ouverture (13) pour insérer le tuyau souple dans la pompe à tuyau souple (1) et d'une seconde ouverture (15) espacée de celle-ci et pour sortir le tuyau souple de la pompe à tuyau souple (1), **caractérisée en ce que**
- une section (17) de la pompe à tuyau souple (1), ou du stator (3), formée entre la première ouverture (13) et la seconde ouverture (15) présente une zone ou un épaulement (19) dans la région de la seconde ouverture (15) qui est plus proche de l'axe de rotation (26) du rotor (5) que toutes les autres sections du stator (3),
- dans laquelle la zone ou l'épaulement (19) ne se situe pas sur la trajectoire d'éléments de déplacement ou des rouleaux (11) de la pompe à tuyau souple (1) ou de son rotor (5).

2. La pompe à tuyau souple (1) selon la revendication 1, dans laquelle les parties du stator (3) entourant ou confinant le rotor (5) ou la section (17) formée entre la première ouverture (13) et la seconde ouverture (15) dépasse(ent) ou tout au moins recouvre(ent) le rotor (5) dans une direction parallèle à l'axe de rotation (26) du rotor (5).

3. La pompe à tuyau souple (1) selon la première ou la seconde revendication, dans laquelle la zone ou l'épaulement (19) présentant une plus petite distance à l'axe de rotation (26) du rotor (5) se situe dans une région de la section (17) formée entre la première et la seconde ouverture (13, 15) espacées l'une de l'autre et ne se situe pas sur la trajectoire de dispositifs de déplacement de fluides du rotor (5) lors l'utilisation de la pompe à tuyau souple (1).

4. La pompe à tuyau souple (1) selon l'une au moins des revendications 1 à 3, dans laquelle la zone ou l'épaulement (19) présentant une plus petite distance à l'axe de rotation (26) du rotor (5) rétrécit vers son extrémité ou sa pointe dans un plan perpendiculaire à l'axe de rotation (26) et dans une direction opposée à la direction de rotation prévue du rotor (5).

5. La pompe à tuyau souple (1) selon au moins l'une des revendications précédentes, dans laquelle le rotor (5) comprend une protection de rotor (7) rotative tournant avec celui-ci pendant utilisation, et dans laquelle la zone ou l'épaulement (19) présentant une plus petite distance à l'axe de rotation (26) du rotor (5) est disposé(e) par rapport à la pompe à tuyau souple (1) de façon à ce que la largeur d'une fente (22) se situant entre la protection de rotor tournante (7) et la zone ou l'épaulement (19) présentant la plus petite distance ne dépasse pas une largeur prédéterminée.

6. La pompe à tuyau souple (1) selon la revendication 5, dans laquelle la largeur prédéterminée de la fente (22) est toute largeur plus petite que toute distance existant entre d'autres sections du stator (3) et le rotor (5) dans un plan perpendiculaire à l'axe de rotation (26).

7. La pompe à tuyau souple (1) selon la revendication 5 ou 6, dans laquelle la largeur prédéterminée de la fente (22) est toute largeur plus petite qu'un diamètre d'un tuyau souple utilisé, ou destiné à être utilisé, avec la pompe à tuyau souple (1) avant ou pendant son utilisation.

8. La pompe à tuyau souple (1) selon l'une des revendications 5, 6 ou 7, dans laquelle la largeur prédéterminée de la fente (22) est toute largeur plus petite qu'une distance moyenne (A) entre une trajectoire circulaire (51) décrite par des dispositifs de déplacement de fluides du rotor (5) lors de la rotation du rotor (5) et un diamètre interne (31) moyen du stator (3).

9. La pompe à tuyau souple (1) selon l'une au moins des revendications précédentes, dans laquelle le rotor (5) comporte une plaque de base (23) aplatie, coupée ou découpée dans l'une au moins de ses sections afin de permettre un retrait du rotor (5) du stator (3) dans la direction de l'axe de rotation (26) du rotor (5).

10. La pompe à tuyau souple (1) selon l'une au moins des revendications précédentes, dans laquelle la section (17) formée entre la première et la seconde ouverture (13, 15) espacées l'une de l'autre, ou un segment de celle-ci, est formée dans une région de la première ouverture (13) pour l'insertion du rotor (5) de façon à rétrécir vers son extrémité ou sa pointe dans un plan perpendiculaire à l'axe de rotation (26) dans une direction de rotation prévue du rotor (5).

11. Dispositif de traitement du sang (24) comprenant au moins une pompe à tuyau souple (1) selon l'une au moins des revendications 1 à 10.

12. Dispositif de traitement du sang (24) selon la revendication 11 configuré comme dispositif d'hémodialyse, dispositif d'hémofiltration, dispositif d'hémodiafiltration, dispositif d'aphérèse, dispositif de transfusion ou comme dispositif d'oxygénation.
